# EUROPEAN PATENT APPLICATION

(11) **EP 1 312 390 A2**
(43) Date of publication of application: **21.05.2003**
(21) Application number: 02257728.2
(22) Date of filing: 07.11.2002
(51) Int. Cl.: A61N 1/00, H01G 9/042, A61N 1/38, A61N 1/39

(54) **Optimal reform protocols for groups IVB and VB electrolytic capacitors**

(30) Priority: 07.11.2001 US 345190 P
(71) Applicant: Wilson Greatbatch Technologies, Inc., Clarence, New York 14031 (US)
(72) Inventor: Liu, Yanming, Clarence Center, New York 14032 (US); Nesselbeck, Neal N., Lockport, New York 14094 (US); Spaulding, Joseph E., Williamsville, New York 14221 (US); Muffoletto, Barry C., Alden, New York 14004 (US)
(74) Representative: Colmer, Stephen Gary

(57) **Abstract**

The present invention relates to a reform protocol to maintain one or more device performance parameters above certain values, and/or below certain values, and/or within certain ranges of values while optimizing battery consumption. In particular, the reform protocol requires:
(1) energizing the valve metal capacitor to a desired energy level, and
(2)
   (a) immediately disconnecting the valve metal capacitor from the energizing source and any external load so the energy in the capacitor is dissipated due to self-discharge, or
   (b) immediately connecting the valve metal capacitor to a non-therapeutic load.

## Description

### CROSS-REFERENCE TO RELATED APPLICATION

This application claims priority to U.S. provisional patent application serial no. 60/345,190, filed on November 7, 2001.

### BACKGROUND OF THE INVENTION

### 1. Field of the Invention

The present invention relates to capacitors, and in particular, those capacitors containing Group IVB and VB elements -- which are tantalum, niobium, titanium, hafnium, vanadium, and zirconium and which are collectively referred to as "valve metal" -- with a liquid electrolyte. Such valve metal capacitors, in particular tantalum capacitors, can be used in many applications that require a capacitor. For this document, however, we will concentrate on such valve metal, in particular tantalum, capacitors that are used in medical devices, such as implantable defibrillators, cardioverters, pacemakers, and more particularly protocols for reforming valve metal capacitors.

### 2. Prior Art

It has been cited in U.S. patent no. 6,283,985 to Harguth et al., that "since the early 1980s, thousands of patients prone to irregular and sometimes life threatening heart rhythms have had miniature defibrillators and cardioverters implanted in their bodies. These devices detect onset of abnormal heart rhythms and automatically apply corrective electrical therapy, specifically one or more bursts of electric current, to hearts. When the bursts of electric current are properly sized and timed, they restore normal heart function without human intervention, sparing patients considerable discomfort and often saving their lives.

The typical defibrillator or cardioverter includes a set of electrical leads, which extend from a sealed housing into the walls of a heart after implantation. Within the housing are a battery for supplying power, a capacitor for delivering bursts of electric current through the leads to the heart, and monitoring circuitry for monitoring the heart and determining when, where, and what electrical therapy to apply. The monitoring circuitry generally includes a microprocessor and a memory that stores instructions not only dictating how the microprocessor answers therapy questions, but also controlling certain device maintenance functions, such as maintenance of the capacitors in the device.

The capacitors are typically aluminum electrolytic capacitors. This type of capacitor usually includes strips of aluminum foil and electrolyte-impregnated paper. Each strip of aluminum foil is covered with an aluminum oxide which insulates the foils from the electrolyte in the paper. One maintenance issue with aluminum electrolytic capacitors concerns the degradation of their charging efficiency after long periods of inactivity. The degraded charging efficiency, which stems from instability of the aluminum oxide in the liquid electrolyte, ultimately requires the battery to progressively expend more and more energy to charge the capacitors for providing therapy.

Thus, to repair this degradation, microprocessors are typically programmed to regularly charge and hold aluminum electrolytic capacitors at or near a maximum-energy voltage (the voltage corresponding to maximum energy) for a time period less than one minute, before discharging them internally through a non-therapeutic load. (In some cases, the maximum-energy voltage is allowed to leak off slowly rather being maintained.) These periodic charge-hold-discharge cycles for maintenance are called "reforms." Unfortunately, the necessity of reforming aluminum electrolytic capacitors reduces battery life.

To eliminate the need to reform, manufacturers developed wet-tantalum capacitors. Wet-tantalum capacitors use tantalum and tantalum oxide instead of the aluminum and aluminum oxide of aluminum electrolytic capacitors. Unlike aluminum oxide, tantalum oxide is reported to be stable in liquid electrolytes, and thus to require no energy-consuming reforms. Moreover, conventional wisdom teaches that holding wet-tantalum capacitors at high voltages, like those used in conventional reform procedures, decreases capacitor life. So, not only is reform thought unnecessary, it is also thought to be harmful to wet-tantalum capacitors."

Harguth et al. claim they "discovered through extensive study that wet-tantalum capacitors exhibit progressively worse charging efficiency over time. Accordingly, there is a previously unidentified need to preserve the charging efficiency of wet-tantalum capacitors.... [Harguth et al.] devised methods of maintaining wet-tantalum capacitors in implantable medical devices. One exemplary method entails reforming this type of capacitor. More particularly, the exemplary method entails charging wet-tantalum capacitors to a high voltage and keeping the capacitors at a high voltage for [a predetermined time period of] about five minutes, before discharging them through a non-therapeutic load. In contrast to conventional thinking, reforming wet-tantalum capacitors at least partially restores and preserves their charging efficiency. Another facet of the invention includes an implantable medical device, such as defibrillator, cardioverter, cardioverter-defibrillator, or pacemaker, having one or more wet-tantalum capacitors and means for reforming the capacitors."

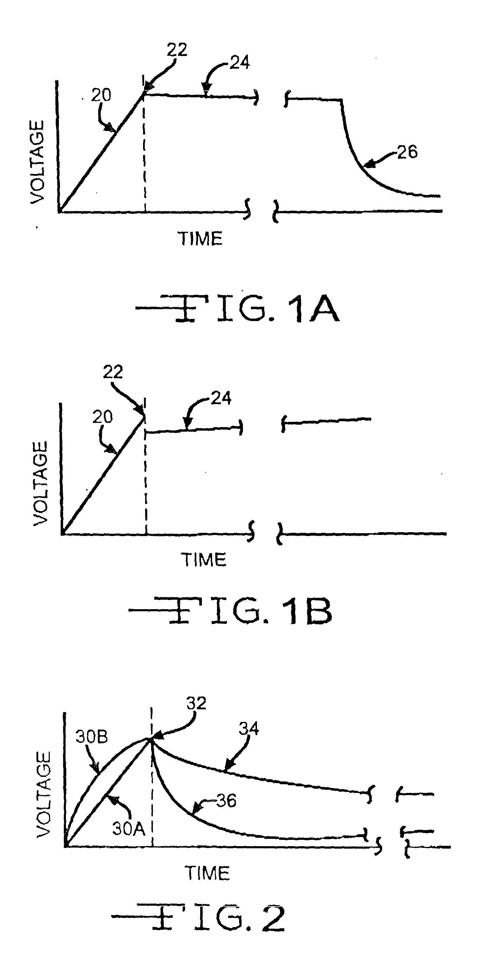

In other words, Harguth et al. disclose, as illustrated in Figure 1, its reform protocol as:
(A) energizing (or referred to as "charging" as in Harguth et al.'s above-identified U.S. Patent and U.S. published patent application no. 2002/0095186 A1) a tantalum capacitor (line 20) to at or below the rated voltage of the tantalum capacitor (point 22);
(B) then maintaining (or as Harguth et al., in the published application, incorrectly define as "charging" by topping off the capacitors) that voltage (line 24) on the capacitor for a predetermined time frame; and
(C) alternatively, connecting the capacitor to a non-therapeutic load (line 26).
The predetermined time frame is between 15 seconds and 10 minutes, and is desired to be around 5 minutes.

Based on this information, it is obvious Harguth et al. obtained patent protection for a method to reform wet-tantalum capacitors when that method was well known for reforming aluminum electrolytic capacitors. It is also quite evident that Harguth et al. knew there were other methods to reform these other electrolytic capacitors -- the maximum-energy voltage is allowed to decline due to self discharge rather than being maintained. Harguth et al., however, failed to discuss or claim these other methods in U.S. Patent no. 6,283,985 as acceptable protocols for reforming valve metal, in particular wet-tantalum, capacitors. Since Harguth et al. unquestionably knew about these other methods and failed to teach, suggest or disclose these other methods for reforming valve metal capacitors, Harguth et al. clearly taught that these other methods are unacceptable for reforming valve metal capacitors.

The present inventors have found Harguth et al.'s reform protocol
(1) to be inefficient by wasting valuable energy,
(2) has a non-optimal charge time for the patient, and
(3) has a susceptibility for the tantalum in the capacitor to have field recrystalization -- a deleterious result caused by maintaining the voltage for too long a time.
The inventors have solved these problems by the invention, which is described below.

### SUMMARY OF THE INVENTION

The present invention relates to a reform protocol to maintain one or more device performance parameters above certain values, and/or below certain values, and/or within certain ranges of values while optimizing battery consumption. In particular, the reform protocol requires:
(1) energizing the valve metal capacitor to a desired energy level, and
(2) (a) immediately disconnecting the valve metal
   capacitor from the energizing source and any external load so the energy in the capacitor is dissipated due to self-discharge, or
   (b) immediately connecting the valve metal capacitor to a non-therapeutic load.

### BRIEF DESCRIPTION OF THE DRAWINGS

Figures 1a and b are graphs illustrating the prior art's reform protocols of wet-tantalum capacitors.

Figure 2 is a graph illustrating the present invention's reform protocol of valve metal capacitors.

Figure 3 is a graph comparing the average cumulative energy consumed during the reforming of valve metal capacitors reformed by the protocols illustrated in Figures 1 and 2.

Figure 4 is a graph comparing the average energy consumed per reform of valve metal capacitors reformed by the protocols illustrated in Figures 1 and 2.

Figure 5 is a graph comparing the average energy efficiencies of valve metal capacitors reformed by the protocols illustrated in Figures 1 and 2.

Figure 6 is a graph illustrating the average charge times for valve metal capacitors that were reformed using different energizing currents in accordance with the protocol illustrated in Figure 2.

Figure 7 is a graph illustrating the average energy efficiencies for valve metal capacitors that were reformed using different energizing currents in accordance with the protocol illustrated in Figure 2.

Figures 8a-e are graphs illustrating the average energy efficiencies of valve metal capacitors that were reformed using different energizing currents to different reform voltages with the reform protocols applied at different frequencies.

### DETAILED DESCRIPTION OF THE INVENTION

The present invention is directed to reform protocols that reduce the energy to charge a valve metal capacitor and maintain a high-energy efficiency. This invention is not directed to valve metal capacitors or the energizing source. For this disclosure, which is not to limit the scope of the present invention, the valve metal capacitors were obtained from Wilson Greatbatch Technologies, Inc. located in Clarence, New York -- the present assignee of this document -- and the energizing source could be purchased from Keithley Instruments of Cleveland, Ohio. Accordingly, the inventors admit those devices are prior art. Instead and as stated above, the present invention is directed to reform protocols that generate superior results for reformed valve metal capacitors.

The present reform protocol, as illustrated in Figure 2, calls for
(A) energizing (line 30a or 30b) a valve metal capacitor to a desired energy level (point 32) and
(B) then immediately (i) disconnecting the valve metal capacitor from the energizing source and any external loads so the energy in the valve metal capacitor dissipates due to self-discharge (line 34) or, alternatively, (ii) connecting the valve metal capacitor to a non-therapeutic load (line 36).

The desired energy level can be the rated voltage of the specific valve metal capacitor, below the rated voltage, above the rated voltage, or a predetermined coulomb level. If the coulomb level is used, then line 30 illustrated in Figure 2 is not at the same angle as illustrated due to losses caused by parasitic and faradaic issues -- which are known to those of ordinary skill in the art. As suggested above, the rated voltage is dependent on the type and make of the valve metal capacitor. Hence, we are unable to provide a definite value for the rated voltage in this document, but those of ordinary skill in the art will understand because different valve metal capacitors can have different rated voltages.

A non-therapeutic load is any active or passive component, or combination thereof, that will discharge and/or de-energize the valve metal capacitor.

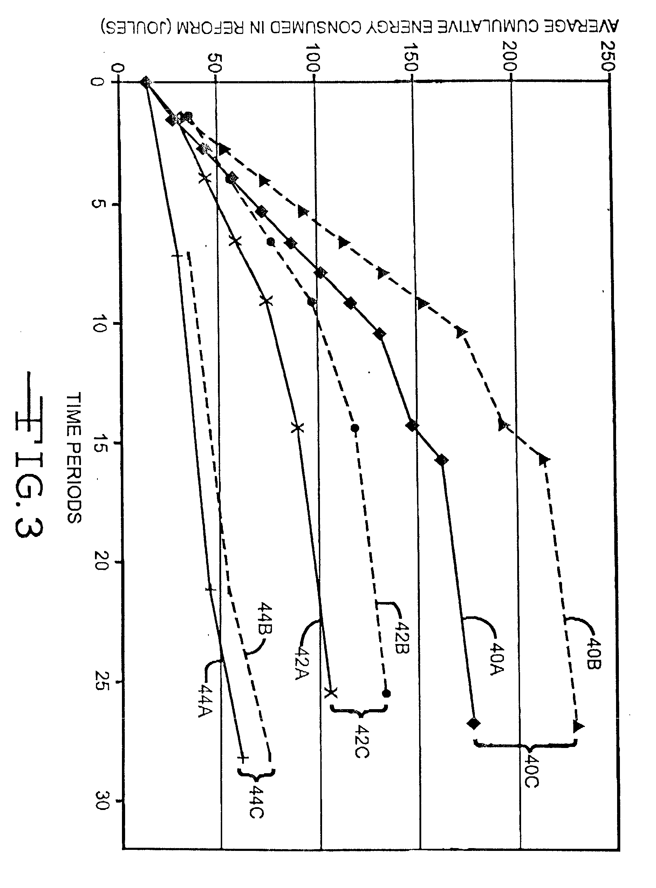

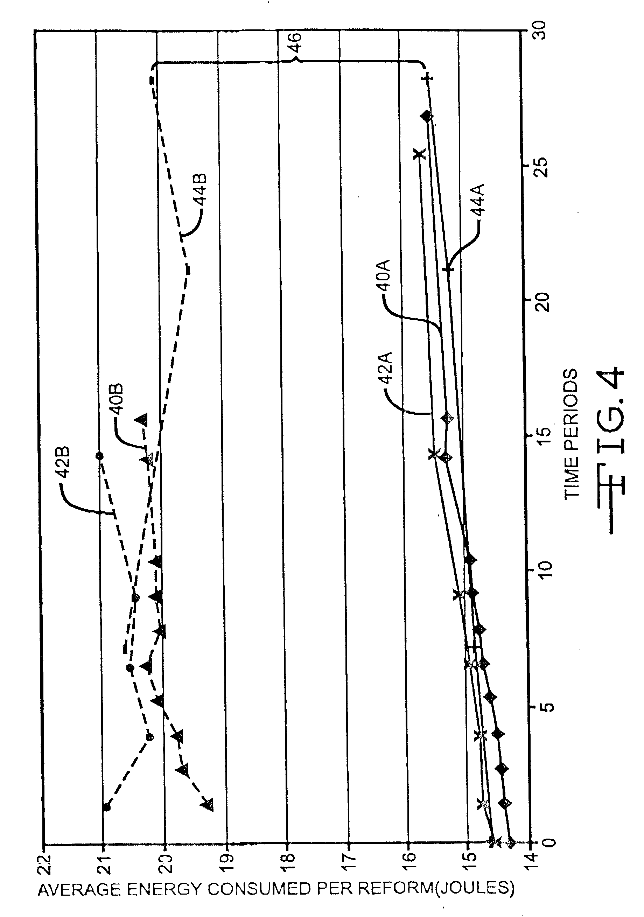

Even those of ordinary skill in the art may not appreciate the significant differences between the reform protocols illustrated in Figures 1 and 2. Hence we need to review Figures 3 and 4 to understand these significant differences.

Figure 3 is a graph illustrating the cumulative energy used by a valve metal capacitor using (A) the reform protocol of the present invention (Figure 2) and (B) Harguth's reform protocol (Figure 1). To prepare this graph, the inventors took twenty-four valve metal capacitors, and divided them into two groups. The first group of capacitors was reformed in accordance with the present invention -- Group A --, and the second group was reformed in accordance with Harguth's reform protocol -- Group B. Each group was then divided into three sub-groups. The first sub-group used a one-month cycle of being charged to rated voltage (lines 40), the second sub-group used a three-month cycle (lines 42), and the third sub-group used a six-month cycle (lines 44).

As illustrated in Figure 3, each sub-group in Group A (lines 40A, 42A, and 44A) used significantly less energy than the corresponding sub-group in Group B (lines 40B, 42B, and 44B). The brackets identified as 40C, 42C, and 44C illustrate the differential in energy for each respective sub-group. Accordingly, it is quite evident Harguth's reform protocol expends energy during the maintaining period that has been found by the inventors to be unnecessary. Hence, the present invention illustrates its superiority by reducing the energy necessary for reforming a valve metal capacitor in relation to Harguth's method.

Figures 4 and 3 are similar but each has a different analysis. Instead of measuring the cumulative energy used in a valve metal capacitor, Figure 4 measures the average energy used in a valve metal capacitor using (A) the reform protocol of the present invention (Figure 2) and (B) Harguth's reform protocol (Figure 1). Since the method of obtaining the data of Figures 3 and 4 are identical, we will not repeat it. However, the differences in efficient energy use between the two protocols are stark and are illustrated by bracket 46. This difference highlighted by bracket 46 clearly illustrates that Harguth's reform protocol expends energy during the maintaining period that has been found by the inventors to be unnecessary. Hence, the present invention illustrates its superiority by reducing the energy necessary for reforming a valve metal capacitor in relation to Harguth's method.

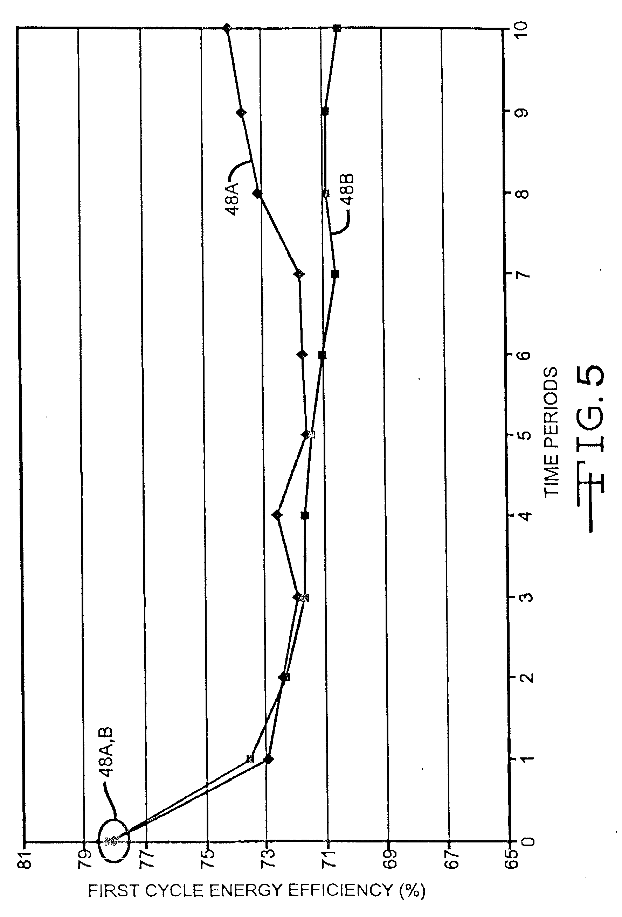

Figure 5 provides further evidence of the superiority of the present invention over Harguth's method. Figure 5 is a comparison of the variations of the first cycle energy efficiency over time. As illustrated, a valve metal capacitor that uses the reform protocol of the present invention (line 48A) has a substantial energy savings over time when compared to a valve metal capacitor that uses the Harguth reform protocol (line 48B). Hence, Figure 5 illustrates that the inventors' reform protocol provides higher energy efficiency than Harguth's reform protocol. That means, a valve metal capacitor that uses the inventors' reform protocol requires less energy to operate and be reformed than the same capacitor that uses the Harguth reform protocol.

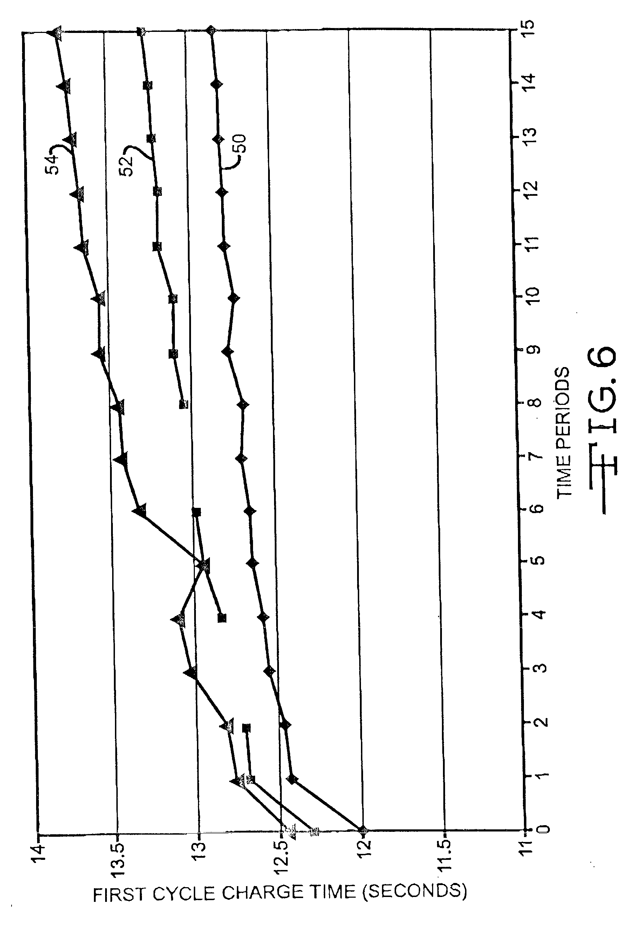

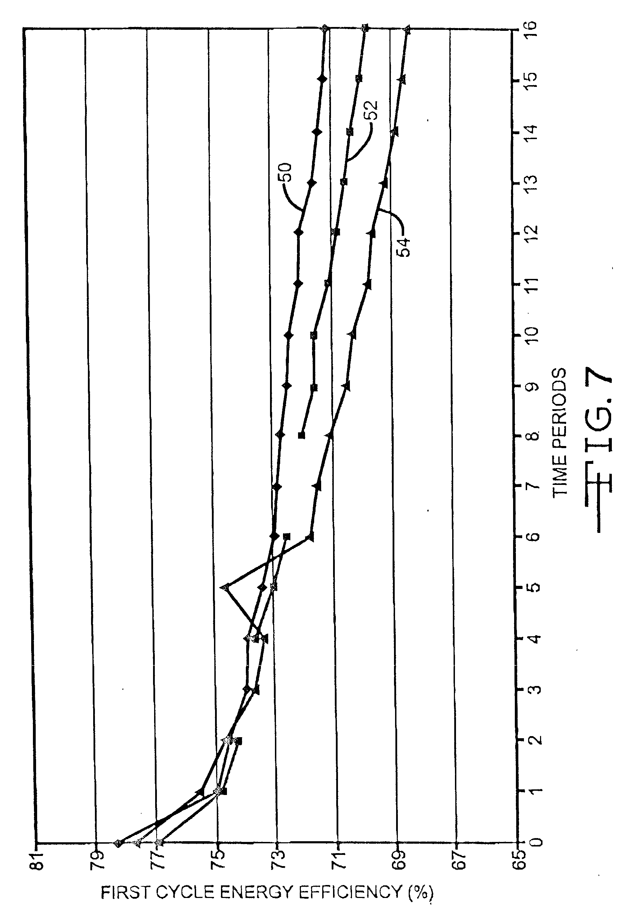

The present inventors also found that charging a valve metal capacitor with a lower current, which lower current is greater than the capacitor's leakage current, results in higher energy efficiency and shorter charge times over the capacitor's life. This analysis can be confirmed by reviewing Figures 6 and 7.

Figures 6 and 7 respectively illustrate the measurements of (a) the average charge times of valve metal capacitors over time and (b) the average energy efficiencies of valve metal capacitors over time. In particular, Figures 6 and 7 show the results of three groups of at least three valve metal capacitors that were reformed at three distinct energizing currents -- line 50 is at 0.25 mA, line 52 is at 0.5 mA, and line 54 is at 1 mA. As seen in these figures, lower reform energizing current results in greater energy efficiency and lower charge time.

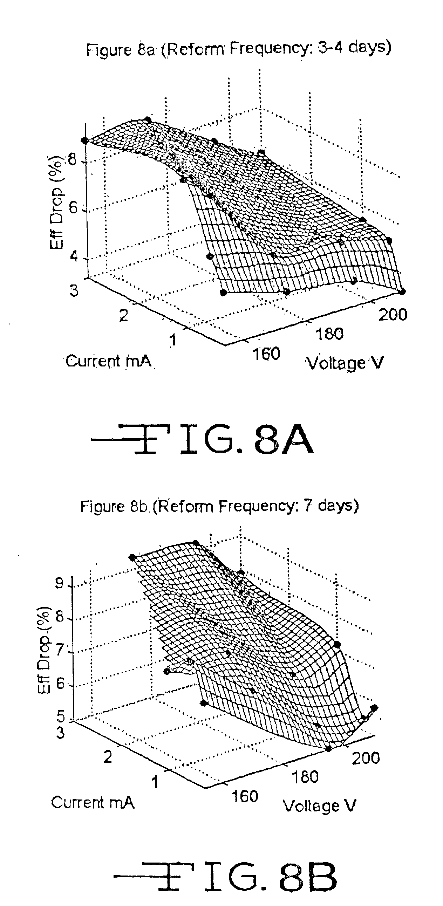

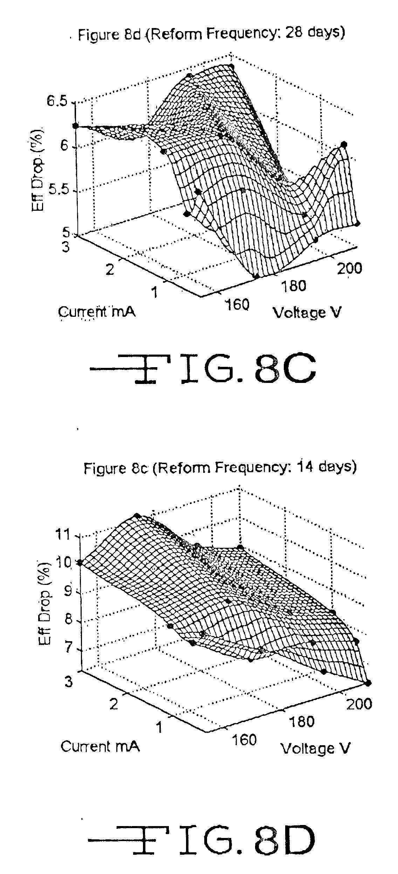

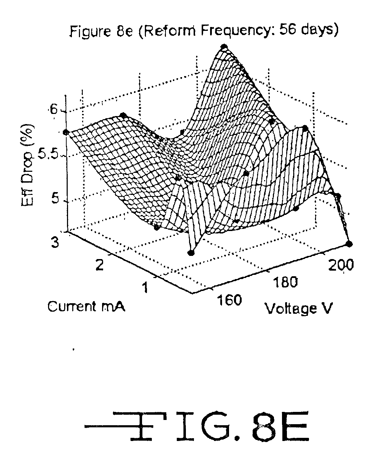

An alternative method to the present reform protocol entails manipulating the reform energizing voltage and the reform energizing current to obtain the desired energy efficiency of the valve metal capacitor to be used with a particular battery. In other words, this document teaches that a user can determine the optimal reform protocol that conforms to Figures 2-7, to maintain one or more device performance parameters above certain values and/or below certain values and/or within certain ranges of values to optimize the battery consumption. An example of such optimization is illustrated in Figures 8a-e. These figures illustrate how different reform energizing currents and reform energizing voltage can alter the average energy efficiencies of valve metal capacitors with the reform protocols applied at different frequencies.

In furtherance of the art, the inventors have discovered an improved method to reform valve metal capacitors in implantable medical devices, and non-medical devices that could use valve metal capacitors, in particular tantalum capacitors.

The embodiments described above are intended only to illustrate and teach one or more ways of practicing or implementing the present invention, not to restrict its breadth or scope. Only the following claims and their equivalents define the actual scope of the invention, which embraces all ways of practicing or implementing the teachings of the invention.

## Claims

1. A method of reforming valve metal (Group IVB and VB elements) capacitors in an implantable medical device, with each capacitor having a rated voltage or a maximum-energy voltage, the method comprising:
energizing the valve metal capacitor to a desired energy level and
immediately disconnecting the valve metal capacitor from the energizing source and any external load so the energy in the valve metal capacitor is dissipated due to self-discharge.

2. A method of reforming valve metal capacitors in an implantable medical device, with each valve metal capacitor having a rated voltage or a maximum-energy voltage, the method comprising:
energizing the valve metal capacitor to a desired energy level and
immediately connecting the valve metal capacitor to a non-therapeutic load.

3. A method of maintaining one or more device performance parameters above certain values, and/or below certain values, and/or within certain ranges of values while optimizing battery consumption comprising:
energizing a valve metal capacitor to a desired energy level and
immediately disconnecting the capacitor from the energizing source and any external load so the energy in the valve metal capacitor is dissipated due to self-discharge or immediately connecting the valve metal capacitor to a non-therapeutic load.

4. The method of Claim 2 or 3 wherein the non-therapeutic load is any active or passive component, or combinations thereof, that will discharge or de-energize the valve metal capacitor

5. The method of any one of the preceding claims wherein the desired energy level is the rated voltage.

6. The method of any one of the preceding claims 1 to 4 wherein the desired energy level is below the rated voltage.

7. The method of any one of the preceding claims 1 to 4 wherein the desired energy level is above the rated voltage.

8. The method of any one of the preceding claims wherein the desired energy level is a predetermined coulomb value.

9. The method of any one of the preceding claims wherein the energizing of the valve metal capacitor has a reform energizing current at least greater than the leakage current of the valve metal capacitor.

10. The method of any one of the preceding claims of further selecting the energizing current of optimum value to obtain a desired energy efficiency of the valve metal capacitor and/or a desired charge time of the valve metal capacitor.

11. The method of any one of the preceding claims wherein the valve metal capacitor is never maintained at any voltage.

12. The method of Claim 11 wherein by not maintaining any voltage during the method of reforming the valve metal capacitor, the valve metal capacitor obtains maximum energy efficiency and the method of reforming valve metal capacitor saves energy.

13. The method of any one of the preceding claims wherein the valve metal is selected from the group consisting of tantalum, niobium, titanium, zirconium, hafnium and vanadium.
